# EUROPEAN PATENT SPECIFICATION

(11) **EP 0 553 960 B1**
(45) Date of publication and mention of the grant of the patent: **27.08.1997**
(21) Application number: 93300160.4
(22) Date of filing: 11.01.1993
(51) Int. Cl.: A61F 2/06, A61M 25/10

(54) **Protective membrane for stent-carrying ballon catheter**
Schützmembran für Stent-tragenden Ballonkatheter
Membrane de protection pour cathéter à ballonnet portant une "stent"

(30) Priority: 31.01.1992 US 828705
(43) Date of publication of application: 04.08.1993
(73) Proprietor: ADVANCED CARDIOVASCULAR SYSTEMS, INC., Santa Clara California 95052 (US)
(72) Inventor: Lau, Lilip, Cupertino, California 95014 (US); Hyde, Gregory M., Sunnyvale, California 94087 (US); Hartigan, William M., Fremont, California 94555 (US); Williams, Michael S., Cupertino, California 95014 (US)
(74) Representative: Mayes, Stuart David

(56) References cited:
- EP-A- 0 292 587
- EP-A- 0 428 479
- EP-A- 0 461 474

## Description

The invention relates generally to a stent delivery system of the type used in conjunction with a balloon or dilatation catheter in, for example, percutaneous transluminal coronary angioplasty (PTCA) procedures, the balloon or dilation catheter having an elastic or expandable covering.

In typical PTCA procedures, a guiding catheter is percutaneously introduced into the cardiovascular system of a patient through the brachial or femoral arteries and advanced through therein until the distal end thereof is in the ostium of the desired coronary artery. A guidewire and a dilatation catheter having a balloon on the distal end thereof are introduced through the guiding catheter with the guidewire sliding within the dilatation catheter. The guidewire is first advanced out of the guiding catheter into the patient's coronary vasculature and the dilatation catheter is advanced over the previously advanced guidewire until the dilatation balloon is properly positioned across the lesion. Once in position across the lesion, the flexible, expandable, preformed balloon is inflated to a predetermined size with radiopaque liquid at relatively high pressures (e.g., greater than about 4 atmospheres) to radially compress the atherosclerotic plaque of the lesion against the inside of the artery wall and thereby dilate the lumen of the artery. The balloon is then deflated to a small profile, so that the dilatation catheter can be withdrawn from the patient's vasculature and blood flow resumed through the dilated artery.

In angioplasty procedures of the kind referenced above, there may be restenosis of the artery, which either necessitates another angioplasty procedure, a surgical bypass operation, or some method of repairing or strengthening the area. To prevent restenosis and strengthen the area, a physician can implant an intravascular prosthesis for maintaining vascular patency, called a stent, inside the artery at the lesion. The stent is expanded to a larger diameter, often by the balloon portion of the catheter. Stents delivered to a restricted coronary artery, expanded to a larger diameter by a balloon catheter, and left in place in the artery at the site of a dilated lesion are shown in U.S. Patent Nos. 4,740,207 (Kreamer) and 5,007,926 (Derbyshire).

The present invention is directed to a stent delivery stem comprising a balloon dilation catheter having an elastic or expandable membrane covering the balloon and which aids in the delivery of a stent by the inflation of the balloon portion. The present invention is not limited, however, to PTCA procedures, but has broader applications, and can be used with any type of stent delivered to any vessel or lumen.

The elastic or expandable membrane reinforces the balloon portion of the dilatation catheter. The catheter with the reinforced balloon portion is then made more suitable for delivery of a stent. The membrane is interspaced between the balloon and the stent, in the form of an elastic tubular sheath affixed to the outside of the balloon.

There are several problems associated with balloon catheter delivered stents that are alleviated with the present invention. For example, by being between the stent and the balloon portion of the catheter, the membrane prevents puncture of the balloon by any protuberance or irregularity found on the stent.

Furthermore, the membrane may be (but is not necessarily) elastic, and the elastic membrane allows the stent to be expanded in a uniform manner. The elastic membrane distributes forces evenly over a larger area, so that a portion of a stent less resistant to radial forces will not expand more than a more resistant portion, as is sometimes the case.

In addition, the membrane prevents damage to artery walls in the event the balloon develops a leak, which causes high pressure fluid to escape through the leak, known as "pin-holing." The membrane would form a protective barrier to minimize the harmful effects of pin-holing in the event the balloon is punctured.

Yet another advantage is that when the membrane is made of an elastic material it provides a substrate for the stent, so the stent can be secured onto the balloon in a more positive manner. The membrane provides a cushion in which the stent can dig into, and provides more friction for the stent than the slippery surface of a balloon, which may be covered with an anti-friction material such as Microglide™ coating, marketed by Advanced Cardiovascular Systems, Inc., (ACS) of Santa Clara, California.

Another advantage of the membrane is that it allows for a decrease in deflation time when the balloon is deflated, after the stent is in place. In one embodiment in which the membrane is made of an elastic material, it reduces the deflation time by squeezing the balloon so it will deflate faster.

Yet another advantage is that the membrane ensures that the balloon will be deflated into a uniform, round balloon, and not into an undesirable flat or pancake form, known as "balloon winging." Balloon winging is undesirable because it increases the likelihood that the balloon will entangle when it is withdrawn from the stent and through the coronary arteries.

A further advantage of the membrane is to prevent the "dog bone" shape that stents are sometimes expanded into by a balloon catheter, when not using the membrane, which results when there is less resistance to the expansion of a stent at its ends than in its middle. Being preferably longer than the stent, the membrane provides increased resistance to the radial forces applied to the ends of a stent, allowing the stent to expand in a uniform manner.

Yet another advantage of the membrane is that the membrane can be impregnated or made with a radiopaque material so that it may be visible during the angioplasty procedure. This would greatly facilitate the placement of stents, which may otherwise be invisible to the fluoroscopy techniques used in angioplasty.

Still another advantage is that a radiopaque marked membrane can indicate whether or not a stent has been expanded to the proper enlarged diameter.

Furthermore, the membrane may be coated or impregnated with a therapeutic agent to provide a localized drug delivery system.

These and other advantages of the invention will become more apparent from the following detailed description thereof when taken in conjunction with the accompanying drawings:
FIG. 1 is an axial cross-section of one embodiment of the present invention in the form of an elastic membrane outside the balloon portion of a catheter and catheter shaft, in the deflated state of the balloon catheter;
FIG. 2 is an axial cross-section of the elastic membrane of FIG. 1 in its inflated state;
FIG. 3 is a cross-sectional view along the diameter of another embodiment of the elastic membrane, employing several layers.

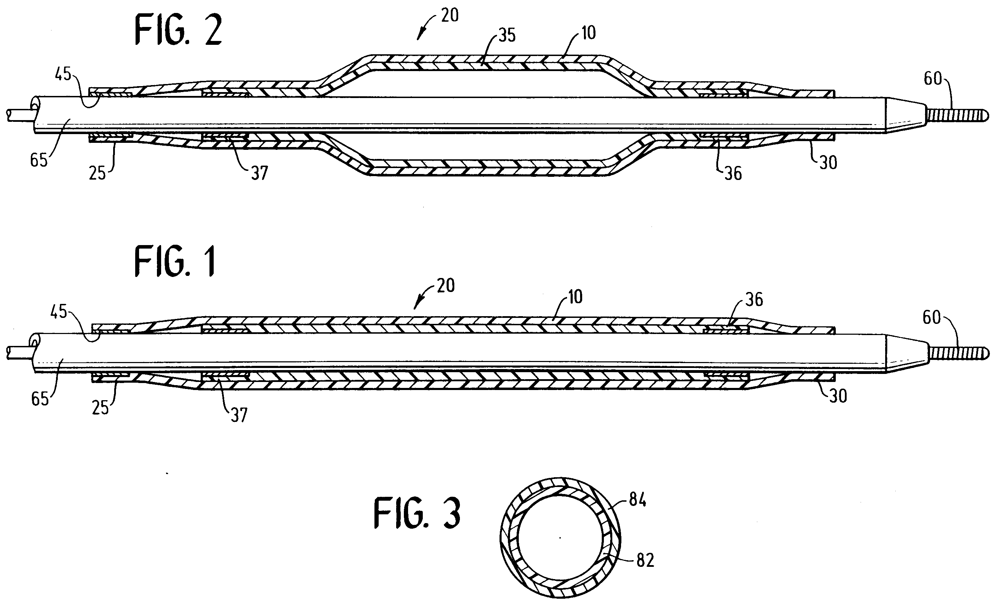

As shown by Figs. 1 and 2, there is shown a first preferred embodiment of the present invention, consisting of an elastic, resilient, membrane layer 10, that forms a sheath or sleeve 20 over the balloon. The membrane layer 10 may be formed of any suitable material that is elastic and resilient. The material preferably is one that has a high degree of linearity (non- plasticity) for a wide range of stress and strain values. In the preferred embodiment, however, any elastic material may be used. Commercially available tubing such as "C-Flex" tubing may be used. "C-Flex" tubing may be obtained from Concept Polymer Technologies of Largo, Florida. In addition, the material should have good tear strength to prevent fracturing or splitting when it is stretched. Suitable materials include silicones, latexes, urethanes, polysiloxane modified styrene-ethylene/butylene-styrene block copolymers (SEBS) and their associated families.

While it is envisioned that in the preferred embodiment of Fig. 1 an elastic material would be used to maximize the benefits of the present invention, it is contemplated that any material could be used, including materials such as the type used to form the balloon portion of a PTCA catheter, like PE-600, a polyethylene based material marketed by Advanced Cardiovascular Systems, Inc. (ACS) of Santa Clara, California. Such materials would be expandable but would not necessarily have to be resilient, as is the material contemplated in the preferred embodiments shown in Figs 1 and 2. Thus, as is known in the art, materials that constitute the balloon portions of PTCA catheters are expandable from one diameter to a larger predetermined diameter, being preformed to expand to the larger diameter, but are not necessarily elastic or resilient.

The material forming the membrane may also be impregnated with a radiopaque marker material, or made with a radiopaque material. Suitable radiopaque materials include iodine based materials and barium salts, including materials containing iodipamide (sold commercially under the trade name Cholografin), iopanoic acid (sold under the trade name Telepaque), barium sulfate, bismuth trioxide, bismuth oxychloride, or powdered metals, such as tantalum.

It is further envisioned that the radiopaque materials in the protective membrane could serve not only to mark the location of the stent in a vasculature, but also to indicate whether or not the stent has been fully expanded to the operational, enlarged diameter at which the stent is properly secured to the body lumen. In conventional expandable stents it is difficult to ascertain when the stent is fully expanded to its operational, enlarged diameter form. Consequently there is always the possibility the stent is under or over expanded. Most stents expanded by balloon catheters rely on the assumption that when the balloon is fully expanded, the stent will by definition be expanded to the proper operational diameter. However, this may not always be the case. It is envisioned that the protective membrane, when containing radiopaque materials, could serve as a visual indicia to indicate when the overlying stent is expanded to its proper operational diameter. This could be accomplished by impregnating or forming the membrane with just enough radiopaque material so that the membrane is only visible to fluoroscopy techniques when the membrane is in a non-operational state, at a diameter less than its proper operational diameter. The membrane would disappear from detection by fluoroscopy when it is fully expanded to the point where the overlying stent would be in its operational, enlarged diameter form. This phenomena can be understood as a consequence of the fluoroscope being able to detect only a certain threshold density of radiopaque material, with density measured in terms of either mass or volume of radiopaque material to surface area of membrane containing such radiopaque material. When the membrane has a radiopaque density greater than or equal to the threshold, the fluoroscope will detect the membrane, but when the density diminishes, such as when the membrane is expanded and its surface area is increased, the fluoroscope will not be able to detect the membrane, and the membrane will disappear from view on the fluoroscope. By varying the amount of radiopaque material in the stent it is possible to achieve a particular density at which the membrane will disappear from view when the membrane is expanded to a diameter at which the overlying stent is in its operational, properly expanded form. Thus a physician would know that when the radiopaque membrane disappears from view, the stent overlying the membrane is at or close to its proper operational diameter, and further expansion of the stent can cease.

As shown in Fig. 2, the membrane layer 10, in the form of a tubular sheath 20 having a proximal end 25 and a distal end 30, surrounds the outside of the balloon portion 35 of a PTCA catheter. Tubular sheath 20 is affixed by adhesive to the catheter at a portion 45 of the catheter shaft or outer member 65, with portion 45 lying at proximal end 25 of the tubular sheath and lying outside the balloon portion 35 of the catheter, which has a proximal portion 37. The balloon portion is affixed to the catheter shaft at proximal portion 37, as is known per se in the art.

It can be seen how sheath 20 entirely overlies and covers the underlying balloon portion 35 of the PTCA catheter. As is known in the art, the balloon portion 35 of the catheter is either bonded to the outer member 65 in an integral manner as shown, or is made one-piece with the outer member. The catheter balloon can be inflated by radiopaque fluid from an inflation port (not shown) extending from a lumen contained in the catheter shaft, or, by other means, such as from fluid communication from a passageway formed between the outside of the catheter shaft and the membrane forming the balloon, depending on the design of the catheter. The details and mechanics of balloon inflation vary according to the design of the catheter, and are known in the art per se.

The balloon portion 35 has a distal end 36 and a proximal end 37 which are both completely covered by tubular sheath 20. As can be seen from the drawings the tubular sheath 20 is affixed to the catheter shaft at portion 45 at a point proximal to proximal end 37 of balloon portion 35, so that the tubular sheath overlies the entire balloon portion of the catheter.

As is shown in the figures, the catheter has an axially extending catheter shaft or outer member (outer lumen) 65, which passes over a guidewire 60. The membrane is adhered to the catheter outer member at a point 45 proximal to the balloon portion of the catheter, rather than distal to the balloon portion, because, in the event of a rupture, the membrane will be fastened at the end upstream from the location of the tear, and thus prevent the membrane from curling or bunching when the catheter is withdrawn. The distal end of the membrane is not secured. This allows for the passage of inflation fluid distal to the balloon in the event of a balloon rupture, through a vent (not shown) in the distal end of the catheter.

Although in the figures the membrane is used with an over-the-wire balloon catheter, it may also be used with any other type of catheter, including fixed wire balloon catheters.

Furthermore, the membrane may be adhered to the catheter not only by adhesive, but also by mechanical means. To this end, the membrane may be adhered by mechanical means that include fasteners made of radiopaque material, such as radiopaque stripes around the proximal end of the membrane, to better define the outer limits of the sheath to a fluoroscope.

Alternatively, the membrane may be placed over the balloon portion without the use of adhesives or mechanical means. Thus it is possible to shrink fit the membrane over the balloon portion, with or without the use of adhesives. For example, one method of shrink fitting the elastic membrane onto a PTCA catheter proceeds as follows: A silicon tube with an inner diameter slightly smaller than the shaft diameter of a catheter is attached to the catheter shaft proximal to the balloon by first immersing the silicone tubing into a Freon™ bath. The silicone absorbs the Freon™ readily and swells in addition to softening. Because of the swelling, the inner diameter of the silicone tubing increases, allowing the tubing to slide over the balloon portion of the catheter. As the Freon™ evaporates from the silicone, the tubing shrinks back to its original dimensions. In doing so, a shrink fit is created between the silicone tubing and the catheter shaft proximal to the balloon.

In addition, it is possible to slide the tubing over the balloon after immersing the tubing in alcohol. It is possible that the alcohol may dissolve some of the lubricating coating often found on a dilatation catheter shaft, such as a Microglide™ Coating, manufactured by Advanced Cardiovascular Systems (ACS) of Santa Clara, California. The alcohol lubricates the sheath during the assembly process, then evaporates readily.

After securing the elastic membrane to the dilatation catheter, a stent is positioned over the membrane. The stent is typically about 15 mm long, while the membrane underneath it would be about 20 mm long. In general, membrane 10 is longer than the balloon portion 35. These dimensions, however, are merely representative and are not meant to be limiting.

The stent is positioned over the membrane and balloon portion of the dilatation catheter and gently crimped onto the membrane, which overlies the balloon, either by hand or with a tool, such as a pair of pliers. The membrane provides a cushion or substrate in which the stent can imbed, to further help secure the stent onto the membrane and thus the balloon portion of the catheter. The elastic membrane is made of a high coefficient of friction material to help the stent hold onto the balloon portion. The balloon catheter is then advanced through and positioned in a patient's vasculature, so that the stent is adjacent to the portion of the vessel where treatment is to take place. The balloon is inflated along with the elastic membrane to expand the stent to an enlarged diameter. During expansion, the sheath provides all of the aforementioned benefits, such as protecting the balloon from rupture, preventing pin- hole effects, preventing distortion of the stent into a "dog bone" shape, providing even expansion of the stent, and securing the stent from axial movement. When the stent has reached the desired diameter, the balloon is deflated. The elastic membrane during deflation provides the aforementioned benefits of reducing deflation time, allowing the balloon to collapse in a uniform manner and preventing the balloon from assuming a "pancake" shape.

In the preferred embodiment, the elastic membrane is designed to lie underneath a stent and over the outside of the balloon catheter. The membrane layer is preferably longer than the stent that covers it, so that the ends of the stent may be uniformly expanded. In addition, the membrane layer may have an inner diameter slightly smaller than the outer diameter of a deflated balloon of a PTCA catheter that the membrane layer overlays, when the diameter of the membrane is measured in its natural state, that is, the state prior to the membrane being fitted over the outside of the deflated balloon of the PTCA catheter. As can be seen from the drawings, the elastic membrane layer expands along with the balloon portion of the PTCA catheter, and is interspaced between the balloon portion and the stent.

Though in the preferred embodiment the elastic material is shown in a tubular shape, the elastic material may also be applied in a band or strip form, to surround the balloon portion of the catheter as a winding. Thus the elastic material may be wound around the balloon portion of the catheter, to form an additional, reinforcing layer to the balloon.

Furthermore, although in the preferred embodiment of Figs. 1 and 2 the elastic membrane is shown as affixed adhesively to the outside of the balloon catheter as a separate entity in the form of a tubular sheath overlaying the balloon, it is also contemplated that the elastic membrane may be totally integrated in a one-piece manner with the catheter. To this end, it is also contemplated that the membrane could be laminated onto the material comprising the balloon of a PTCA catheter, either on the outside surface or even the inside surface of the balloon, to reinforce the balloon. If the elastic membrane is placed on the inside of the layer of material forming the balloon, it would not directly contact the stent, however, many of the benefits attained when the elastic membrane is on the outside of the balloon would still be realized.

Furthermore, the elastic material may be deposited or coated chemically onto the balloon portion, on either the inside or the outside surface.

Turning to Fig. 3, there is shown another design of sheath employing multiple layers of material. An inner layer 82 and an outer layer 84 are co-extruded together to form a multi-layered sheath. Inner layer 82 may be made of an inelastic but expandable material such as PE, PE-600 or PET, while outer layer 84 may be an elastic material, such as C-Flex. There may also be more than two layers, in any order of layering, but preferably the elastic layer or layers overlie the inelastic layer or layers.

In addition, as before, in any of these embodiments any suitable material could be utilized for the membrane, including the same material that constitutes the balloon portion of a catheter.

Furthermore, although the preferred use of the balloon reinforcement is for facilitating the delivery of a stent by a balloon catheter, the reinforced balloon catheter may be used for other uses as well.

## Claims

1. A stent delivery system for delivering a stent of the type used in conjunction with a balloon dilatation catheter to reinforce or repair a vasculature, the stent delivery system comprising:
a balloon dilatation catheter with an elongated tubular member (65) having a proximal and distal end, the tubular member (65) having an inflation lumen extending therein;
a flexible, relatively inelastic balloon (35) located proximal the distal end of the tubular member (65), the balloon (35) having a distal end (36) and a proximal end (37) attached to the tubular member (65), the balloon (35) having an inflation means in communication therewith whereby the balloon (35) may be inflated and deflated;
a stent having an expanded position and a collapsed position; and
a tubular sheath (20) having a proximal end (25), a distal end (30), and a central portion, characterised in that the tubular sheath (20) is disposed about the balloon (35) between the balloon and the stent, the central portion of the tubular sheath having an inner surface coated with a low coefficient of friction material and an outer surface coated with a high coefficient of friction material, the tubular sheath (20) being secured to the balloon dilatation catheter;
the balloon (35) has an outer surface coated with a low coefficient of friction material so that with the inner surface of the tubular sheath (20) the outer surface of the balloon (35) forms a substantially frictionless interface permitting the central portion of the tubular sheath to expand substantially without friction upon balloon inflation and translating uneven radial force created by asymmetrical balloon inflation into uniform radial force upon the collapsed stent, forcing symmetrical expansion of the stent and thereby proper stent installation in the vasculature; and
the collapsed stent is releasably secured to the high friction outer surface of the tubular sheath thereby retaining the stent on the sheath (20) while the stent is advanced through the vasculature until the collapsed stent is expanded and released at the point of its installation within the vasculature.

2. The stent delivery system of claim 1, wherein the tubular sheath (20) comprises an elastic membrane (10) with an axial length greater than the axial length of the stent.

3. The stent delivery system of claim 1, wherein the tubular sheath (20) comprises an elastic membrane (10) which uniformly expands when the balloon (35) is expanded.

4. The stent delivery system of claim 1, wherein the tubular sheath (20) has an inner diameter defined as its diameter when the tubular sheath (20) is in a collapsed state prior to being fitted over the outside of the balloon (35), the inner diameter of the tubular sheath (20) being less than the outer diameter of the balloon when the balloon (35) is in its deflated state.

5. The stent delivery system of claim 1, wherein the tubular sheath (20) is formed of a material selected from the group consisting of silicone, latex, polyethylene or derivatives thereof.

6. The stent delivery system of claim 1, wherein the proximal end (25) of the tubular sheath is adhesively secured to the proximal end (45) of the balloon dilatation catheter.

7. The stent delivery system of claim 1, wherein the tubular sheath (20) is secured to the balloon dilatation catheter by shrink fitting.

## Patentansprüche

1. Stent-Abgabesystem zum Abgeben eines Stents des Typs, welcher in Verbindung mit einem Ballonaufweitungskatheder verwendet wird, um ein Gefäß zu verstärken oder zu reparieren, wobei das Stent-Abgabesystem umfaßt:
einen Ballonaufweitungskatheder mit einem langgestreckten röhrenartigen Element (65) mit einem proximalen und einem distalen Ende, wobei das röhrenartige Element (65) ein sich darin erstreckendes Befülllumen aufweist,
einen flexiblen, relativ inelastischen Ballon (35), welcher nahe dem distalen Ende des röhrenartigen Elements (65) angeordnet ist, wobei der Ballon (35) ein distales Ende (36) und ein proximales Ende (37) aufweist, die an dem röhrenartigen Element (65) angebracht sind, wobei der Ballon (35) ein mit diesem in Verbindung stehendes Befüllmittel aufweist, wodurch der Ballon (35) befüllt und entleert werden kann,
einen Stent, welcher eine aufgeweitete Stellung und eine kollabierte Stellung aufweist, und
eine röhrenartige Umhüllung (20) mit einem proximalen Ende (25), einem distalen Ende (30) und einem zentralen Abschnitt,
dadurch gekennzeichnet,
daß die röhrenartige Umhüllung (20) um den Ballon (35) herum zwischen dem Ballon und dem Stent angeordnet ist, wobei der zentrale Abschnitt der röhrenartigen Umhüllung eine Innenoberfläche aufweist, welche mit einem Material mit geringem Reibungskoeffizienten beschichtet ist, und eine Außenoberfläche aufweist, welche mit einem Material mit hohem Reibungskoeffizienten beschichtet ist, wobei die röhrenartige Umhüllung (20) an dem Ballonaufweitungskatheder festgelegt ist,
daß der Ballon (35) eine Außenoberfläche aufweist, welche mit einem Material mit geringem Reibungskoeffizienten beschichtet ist, so daß die Außenoberfläche des Ballons (35) mit der Innenoberfläche der röhrenartigen Umhüllung (20) einen im wesentlichen reibungslosen Übergang bildet, welcher ermöglicht, daß der zentrale Abschnitt der röhrenartigen Umhüllung sich im wesentlichen ohne Reibung beim Befüllen des Ballons aufweitet und eine ungleichmäßige radiale Kraft, welche durch ein unsymmetrisches Befüllen des Ballons erzeugt wird, in eine gleichmäßige radiale Kraft auf den kollabierten Stent umsetzt, wodurch eine symmetrische Expansion des Stents und dadurch eine geeignete Installation des Stents in dem Gefäß erzwungen wird, und
daß der kollabierte Stent lösbar an der Außenoberfläche der röhrenartigen Umhüllung mit hohem Reibungskoeffizienten gesichert ist, wodurch der Stent an der Umhüllung (20) gehalten ist, während der Stent durch das Gefäß vorgeschoben wird, bis der kollabierte Stent erweitert und am Punkt von dessen Installation innerhalb des Gefäßes freigegeben wird.

2. Stent-Abgabesystem nach Anspruch 1, worin die röhrenartige Umhüllung (20) eine elastische Membran (10) mit einer axialen Länge umfaßt, welche größer ist als die axiale Länge des Stents.

3. Stent-Abgabesystem nach Anspruch 1, worin die röhrenartige Umhüllung (20) eine elastische Membran (10) umfaßt, welche sich gleichförmig aufweitet, wenn der Ballon (35) aufgeweitet wird.

4. Stent-Abgabesystem nach Anspruch 1, worin die röhrenartige Umhüllung (20) einen Innendurchmesser aufweist, welcher als deren Innendurchmesser definiert ist, wenn die röhrenartige Umhüllung (20) vor dem Passen über die Außenseite des Ballons (35) in einem kollabierten Zustand ist, wobei der Innendurchmesser der röhrenartigen Umhüllung (20) kleiner ist als der Außendurchmesser des Ballons (35), wenn der Ballon in seinem entleerten Zustand ist.

5. Stent-Abgabesystem nach Anspruch 1, worin die röhrenartige Umhüllung (20) aus einem Material gebildet ist, das aus der Gruppe ausgewählt ist, die besteht aus: Silikon, Latex, Polyethylen oder Derivate davon.

6. Stent-Abgabesystem nach Anspruch 1, worin das proximale Ende (25) der röhrenartigen Umhüllung anhaftend an dem proximalen Ende (45) des Ballonaufweitungskatheders gesichert ist.

7. Stent-Abgabesystem nach Anspruch 1, worin die röhrenartige Umhüllung (20) an dem Ballonaufweitungskatheder durch Schrumpfpassung gesichert ist.

## Revendications

1. Système d'alimentation en petit tube extensible (stent) pour délivrer un petit tube extensible du type utilisé en relation avec un cathéter à dilatation à ballonnet pour renforcer ou pour réparer un système vasculaire, le système d'alimentation en petit tube extensible comprenant :
un cathéter à dilatation à ballonnet muni d'un élément tubulaire allongé (65) ayant une extrémité proximale et une extrémité distale, l'élément tubulaire (65) ayant un conduit de gonflage s'étendant en son sein ;
un ballonnet souple, relativement non élastique (35) situé à proximité de l'extrémité distale de l'élément tubulaire (65), le ballonnet (35) ayant une extrémité distale (36) et une extrémité proximale (37) fixées à l'élément tubulaire (65), le ballonnet (35) ayant des moyens de gonflage en communication avec ce dernier, de sorte que le ballonnet (35) peut être gonflé et dégonflé ;
un petit tube extensible ayant une position dilatée et une position repliée ; et
une gaine tubulaire (20) ayant une extrémité proximale (25), une extrémité distale (30), et une partie centrale, caractérisé en ce que la gaine tubulaire (20) est disposée autour du ballonnet (35) entre le ballonnet et le petit tube extensible, la partie centrale de la gaine tubulaire ayant une surface intérieure revêtue d'une matière à faible coefficient de friction et une surface extérieure revêtue d'une matière à coefficient de friction élevé, la gaine tubulaire (20) étant solidement fixée au cathéter à dilatation à ballonnet ;
en ce que le ballonnet (35) a une surface extérieure revêtue d'une matière à faible coefficient de friction de sorte qu'avec la surface intérieure de la gaine tubulaire (20), la surface extérieure du ballonnet (35) forme une interface sensiblement sans friction permettant à la partie centrale de la gaine tubulaire de se dilater sensiblement sans friction lors du gonflage du ballonnet, et transformant une force radiale non uniforme créée par un gonflage asymétrique du ballonnet en une force radiale uniforme sur le petit tube extensible replié, provoquant une dilatation symétrique du petit tube extensible et, de ce fait, une installation correcte du petit tube extensible dans le système vasculaire ; et
en ce que le petit tube extensible replié est solidement fixé, de manière amovible, à la surface extérieure à friction élevée de la gaine tubulaire, retenant, de ce fait, le petit tube extensible sur la gaine (20) tandis que le petit tube extensible est avancé à travers le système vasculaire jusqu'à ce que le petit tube extensible replié soit dilaté et libéré au niveau du point de son installation à l'intérieur du système vasculaire.

2. Système d'alimentation en petit tube extensible selon la revendication 1, dans lequel la gaine tubulaire (20) comprend une membrane élastique (10) ayant une longueur axiale plus grande que la longueur axiale du petit tube extensible.

3. Système d'alimentation en petit tube extensible selon la revendication 1, dans lequel la gaine tubulaire (20) comprend une membrane élastique (10) qui se dilate de manière uniforme lorsque le ballonnet (35) est dilaté.

4. Système d'alimentation en petit tube extensible selon la revendication 1, dans lequel la gaine tubulaire (20) a un diamètre intérieur défini comme son diamètre lorsque la gaine tubulaire (20) est dans un état replié avant d'être ajustée sur'l'extérieur du ballonnet (35), le diamètre intérieur de la gaine tubulaire (20) étant inférieur au diamètre extérieur du ballonnet lorsque le ballonnet (35) est dans son état dégonflé.

5. Système d'alimentation en petit tube extensible selon la revendication 1, dans lequel la gaine tubulaire (20) est formée d'une matière sélectionnée parmi le groupe constitué de la silicone, du latex, du polyéthylène ou des dérivés de ceux-ci.

6. Système d'alimentation en petit tube extensible selon la revendication 1, dans lequel l'extrémité proximale (25) de la gaine tubulaire est solidement fixée, de manière adhésive, à l'extrémité proximale (45) du cathéter à dilatation à ballonnet.

7. Système d'alimentation en petit tube extensible selon la revendication 1, dans lequel la gaine tubulaire (20) est solidement fixée au cathéter à dilatation à ballonnet par un ajustement par rétrécissement.
